# EUROPÄISCHE PATENTSCHRIFT

(11) **EP 3 246 303 B1**
(45) Veröffentlichungstag und Bekanntmachung des Hinweises auf die Patenterteilung: **27.11.2019**
(21) Anmeldenummer: 16170274.1
(22) Anmeldetag: 19.05.2016
(51) Int. Cl.: C07C 45/50, C07C 47/02, C07C 5/05, C07C 5/333, C07C 7/163, C07C 11/08, B01J 19/18, B01J 8/04

(54) **HERSTELLUNG VON N-PENTANAL AUS BUTENARMEN EINSATZSTOFFGEMISCHEN**
PREPARATION OF N-PENTANAL FROM LOW-BUTENE REACTION FEED
PRODUCTION DE N-PENTANAL A PARTIR DE MELANGES DE MATIERES DE DEPART PAUVRES EN BUTENE

(43) Veröffentlichungstag der Anmeldung: 22.11.2017
(73) Patentinhaber: Evonik Degussa GmbH, 45128 Essen (DE)
(72) Erfinder: FRANKE, Robert, 45772 Marl (DE); ALTMANN, Lena, 45657 Recklinghausen (DE); HECHT, Corinna, 45721 Haltern am See (DE); DERCKS, Benedikt, 44809 Bochum (DE); SPOHR, Hanna, 47199 Duisburg-Baerl (DE); ZANTHOFF, Horst-Werner, 45481 Mülheim a.d. Ruhr (DE)
(74) Vertreter: Evonik Patent Association

(56) Entgegenhaltungen:
- WO-A1-2004/041763

## Beschreibung

Die Erfindung befasst sich mit der Fragestellung, wie n-Pentanal im Wege der Hydroformylierung aus Einsatzstoffgemischen hergestellt werden kann, die einen geringen Anteil von n-Buten und einen großen Anteil von n-Butan aufweisen. Konkret wird nach Lösungen gesucht, wie etablierte Prozesse zur Hydroformylierung solcher butenarmen Gemische hinsichtlich der stofflichen Verwertung weiter optimiert werden können.

Die in diesem Zusammenhang diskutierten Stoffgruppen sind im Wesentlichen Alkene (Olefine), Alkane (Paraffine), Aldehyde und Alkohole. Diese Begriffe werden hier entsprechend der in der Chemie üblichen Terminologie verwendet.

In der organischen Chemie werden Stoffgruppen gemeinhin nach der Anzahl ihrer Kohlenstoffatome eingeteilt und benannt. Dabei wird der interessierenden Stoffklasse das Präfix Cₙ- vorangestellt, wobei n für die Anzahl der jeweiligen Kohlenstoffatome des Stoffes steht. Ist beispielsweise von C₄-Alkenen die Rede, so sind die vier isomeren Olefine mit vier Kohlenstoffatomen gemeint, nämlich Isobuten, 1-Buten, cis-2-Buten und trans-2-Buten.

Die gesättigten Alkane sind kaum reaktiv und werden deswegen überwiegend als Brennstoff oder Aerosol-Treibstoff verwendet.

Aus den reaktiveren Alkenen lassen sich indes Kohlenwasserstoffe mit einer größeren Anzahl an Kohlenstoffatomen aufbauen, die ein weites Anwendungsspektrum erschließen und damit höhere Verkaufspreise erzielen als die Ausgangsstoffe mit einer geringen Anzahl Kohlenstoffatome. Auf diese Weise schöpft die industrielle organische Chemie Wert.

Eine wirtschaftlich bedeutende Stoffklasse, die aus dieser Motivation aus niederen Alkenen herstellt wird, sind die Aldehyde. Der Aldehyd mit drei Kohlenstoffatomen heißt Propanal. Es existieren zwei C₄-Aldehyde, nämlich n-Butanal und iso-Butanal.

Zu den Aldehyden mit fünf Kohlenstoffatomen gehören die isomeren Stoffe n-Pentanal (auch Valeraldehyd genannt), iso-Pentanal (Iso-Valeraldehyd), (S)-2-Methylbutyraldehyd, (R)-2-Methylbutyraldehyd und tert-Pentanal. Von wirtschaftlichem Interesse ist Valeraldehyd, welcher als Vulkanisationsbeschleuniger verwendet wird. Darüber hinaus kann Valeraldehyd durch Aldolkondensation und anschließender Hydrierung zu 2-Propylheptanol umgesetzt werden, einem Alkohol, welcher wiederum Ausgangsstoff für weitere Synthesen in Richtung PVC-Weichmacher, Detergenzien und Schmiermittel darstellt. Näheres hierzu findet sich in US8581008.

n-Pentanal wird durch Hydroformylierung von n-Buten hergestellt.

N-Buten ist eine Sammelbezeichnung für die drei linearen C₄-Olefine 1-Buten, cis-2-Buten und trans-2-Buten. Meist handelt es sich um ein Gemisch, welches diese drei isomeren Stoffe enthält; die genaue Zusammensetzung hängt vom thermodynamischen Zustand ab.

Unter Hydroformylierung (Oxo-Reaktion) versteht man allgemein die Umsetzung von ungesättigten Verbindungen wie insbesondere Olefinen mit Synthesegas (Wasserstoff und Kohlenmonoxid) in Aldehyde, deren Anzahl an Kohlenstoffatome um eins höher liegt als die Anzahl der Kohlenstoffatome der Ausgangsverbindungen. Zur Herstellung von C₅-Aldehyden wird dementsprechend Buten hydroformyliert.

Eine umfassende Darstellung des derzeitigen Standes der Hydroformylierung findet sich in:
Armin Börner, Robert Franke: Hydroformylation. Fundamentals, Processes and Applications in Organic Synthesis. Volumes 1 and 2. Wiley-VCH, Weinheim, Germany 2016.

Ein etabliertes Verfahren zur Herstellung von n-Pentanal ist aus US9272973 bekannt. Die Erfinder gehen von diesem nächstliegenden Stand der Technik aus.

Bei der dort praktizierten Hydroformylierung zur Herstellung von Valeraldehyd wird ein Einsatzgemisch verwendet, welches 35 % 2-Butene enthält und lediglich 1 % 1-Buten. Der Rest ist Butan, welches sich in der Hydroformylierung inert verhält. Das extrem an 1-Buten arme Gemisch wird in Gegenwart eines homogenen Katalysatorsystems hydroformyliert, welches einen besonderen, symmetrischen Bisphosphit-Liganden umfasst, welcher durch Zugabe eines Amins stabilisiert wird. Als Lösungsmittel wird Isononylbenzoat erwähnt. Mit diesem Katalysatorsystem werden Butenumsätze von 60 bis 75 % erzielt.

Zur Steigerung der stofflichen Effizienz schlägt WO2015/086634A1 vor, die nicht umgesetzten Alkene mit Hilfe einer Membran aus dem Reaktionsgemisch abzutrennen und in einer zweiten Hydroformylierungsstufe mit Hilfe der SILP-Technologie umzusetzen. Die inerten Alkane werden ebenfalls mit der Membran aus dem Prozess ausgeschleust, sodass sie in der Hydroformylierung nicht weiter stören. Durch diese Maßnahme können die im Einsatzstoffgemisch enthaltenen Butene stofflich sehr gut ausgenutzt, dass heißt in Aldehyde umgesetzt werden. Die im Einsatzstoffgemisch enthaltenen Butane bleiben indes ungenutzt.

Eine Möglichkeit Alkane chemisch besser zu verwerten als sie zu verbrennen besteht darin sie zu dehydrieren. Durch Dehydrierung lassen sich Alkane in reaktivere und damit chemisch vielfältig nutzbare Alkene umsetzen. Freilich erfordert dies Energie. Da Alkane vergleichsweise preiswerte Rohstoffe sind, erzielt eine kostengünstig, insbesondere energieeffizient durchgeführte Dehydrierung eine beachtliche Wertschöpfung. Aus diesem Grund gibt es ein ansehnliches Angebot an kommerziell erhältlichen Technologien zur Dehydrierung von Alkanen, genauer gesagt von dem C₃-Alkan Propan. Eine umfassende Marktstudie findet sich in:
Victor Wan, Marianna Asaro: Propane Dehydgenation Process Technologies, October 2015. Erhältlich bei IHS CHEMICAL, Process Economics Program RP267A.

Da die Propan-Dehydrierung im Umfeld von Naphtha-Crackern betrieben wird, sind diese Prozesse allesamt auf einen Durchsatz in petrochemischer Dimension ausgelegt und optimiert. So beträgt die Kapazität einer Propan-Dehydrierung nach dem UHDE STAR® Prozess etwa 500 000 t/a Propylen (siehe vorstehend zitierter PEP-Report Seite 2-11). Dies sind Größenordnungen, die sich sehr deutlich von denen der industriell betriebenen Hydroformylierung unterscheiden; so beträgt die Kapazität einer großen Oxo-Anlage lediglich 100 000 t/a (Börner/Franke, Abschnitt Introduction). Es macht deshalb wirtschaftlich kaum Sinn, Propan mit Hilfe einer kostspieligen Großanlage erst zu Dehydrieren und dann einen kleinen Teil des gewonnen Propens zu hydroformylieren, solange das überschüssige Propen nicht etwa zur Produktion von Polypropylen genutzt wird.

Wohl aus diesem Grunde wird in der Literatur auffällig selten über eine Kombination einer Dehydrierung mit einer anschließenden Hydroformylierung berichtet:
Auf dem Gebiet der C₃-Chemie beschreibt US6914162B2 die Kombination einer Propan-Dehydrierung mit anschließender Hydroformylierung des erhaltenen Propens. Die Dehydrierung ist somit stromaufwärts vor der Hydroformylierung angeordnet. "Stromaufwärts" meint in diesem Zusammenhang in der Wertschöpfungskette vorgelagert. Ein ähnlicher Prozess, der auch mit n-Butan als Einsatzstoff arbeitet, ist in US2006/0122436A1 umrissen.

WO2015/132068A1 beschreibt ebenfalls die Dehydrierung von C₃ bis C₅ Alkanen mit stromabwärts (d.h. in Richtung der Wertschöpfung) angeordneter Hydroformylierung. Allerdings erfolgt letztere in Gegenwart eines heterogenen Katalysatorsystems, weswegen sich dieser Prozess apparativ deutlich von den derzeit industriell betriebenen, homogen katalysierten Oxo-Verfahren unterscheidet.

Ein weiterer Grund dafür, warum die Dehydrierung und die Hydroformylierung in der Praxis nicht kombiniert werden, ist dass bei der Dehydrierung neben den gewünschten Alkenen noch sehr viele andere Kohlenstoffverbindungen entstehen, die in der Hydroformylierung sehr hinderlich sind. Ein Beispiel dafür ist etwa 1.3-Butadien, welches in der Hydroformylierung als Inhibitor wirkt. Solche Störstoffe müssen erst aufwendig (d.h. kostspielig) von den Alkenen abgetrennt werden, bevor diese hydroformyliert werden können.

Die Zwischenabtrennung von bei der Dehydrierung entstehenden, in der Hydroformylierung unerwünschten Stoffen ist in US8889935 thematisiert. Allerdings dient dieses Verfahren primär dazu, durch Dehydrierung von n-Butan das lineare C₄-Olefin 1-Buten herzustellen. In diesem Zusammenhang wird vorgeschlagen, das bei der Dehydrierung von n-Butan als Nebenprodukt anfallende 2-Buten durch Hydroformylierung in n-Pentanal umzusetzen. In der Dehydrierung entstehende Störstoffe wie 1.3-Butadien werden vor der Hydroformylierung derivatisiert bzw. selektiv hydriert.

Schließlich schildert US5998685 ein Verfahren, bei welchem Einsatzstoffgemische enthaltend n-Butan und iso-Butan dehydriert und die dabei erhaltenen Alkene zunächst oligomerisiert und die erhaltenen Olefin-Oligomere schließlich hydroformyliert werden. Da die in der Oligomerisierung eingesetzten Katalysatoren ebenfalls sehr empfindlich auf in der Butan-Dehydrierung anfallende Nebenprodukte reagieren, ist eine aufwändige Reinigung dazwischen geschaltet.

In Hinblick auf diesen Stand der Technik lässt sich sagen, dass aufgrund der Durchsatzmengen-Unterschiede und der zwangsläufig gebildeten Störstoffe die Alkan-Dehydrierung mit anschließender Hydroformylierung keinerlei praktische Bedeutung erlangt hat.

Aber auch die umgekehrte Kombination, bei der die Hydroformylierung stromaufwärts vor der Dehydrierung angeordnet ist, findet sich in der Patentliteratur kaum:
So offenbart MY140652A ein Verfahren zur Herstellung von Oxo-Alkoholen, bei dem die in der Hydroformylierung nicht umgesetzten Alkane aus dem Hydroformylierungsgemisch abgetrennt und einer Dehydrierung unterworfen werden. Die dabei erhaltenen Alkene werden mit dem frischen Einsatzstoff gemischt und vor der Hydroformylierung noch isomerisiert. Der Einsatzstoff stammt aus einem Fischer-Tropsch-Prozess und umfasst im Wesentlichen Alkane und Alkene mit acht bis zehn Kohlenstoffatome.

Bei diesem Prozess werden die Alkane vor der Dehydrierung destillativ aufwändig von in der Hydroformylierung nicht umgesetzten Alkenen abgetrennt. Alkene sind nämlich in der Dehydrierung ungern gesehen, da sie aufgrund ihrer verglichen mit Alkanen bis zu vierfach höheren Reaktivität viele Oxidations-Produkte wie CO und CO₂ bilden, was letztendlich zu einer raschen Verkokung der Katalysatoren führt; vgl. R. Nielsen: Process Economics Program Report 35F On-Purpose Butadiene Production II. December 2014, Seite 39 erhältlich von ihs.com/chemical. Aus demselben Grund raten die Anbieter kommerzieller Dehydrier-Prozesse davon ab, Alkene in die Dehydrierung zu fahren.

Das praktische Problem aufwändig abzutrennender Störstoffe besteht also auch bei der Anordnung einer Dehydrierung stromabwärts einer Hydroformylierung. Zudem wird es selbst mit einer Oxo-Anlage im Weltmaßstab kaum möglich sein, eine Dehydrier-Anlage im üblichen Format nur annähernd auszulasten.

Zusammenfassend lässt sich festhalten, dass die Kombinationen aus Dehydrierung und Hydroformylierung bzw. aus Hydroformylierung und Dehydrierung industriell nicht betrieben wird, da die technischen Größenordnungen nicht übereinstimmen und weil die Spezifikationen der Produkte und Edukte der jeweiligen Prozesse inkompatibel sind und deswegen eine kostspielige Zwischenabtrennung erfordern.

Vor diesem Hintergrund macht es sich die Erfindung zur Aufgabe, ein Verfahren zur Herstellung von n-Pentanal aus Einsatzstoffgemischen mit einem geringen Anteil von n-Buten und einen großen Anteil von n-Butan so weiterzubilden, dass die stoffliche Verwertung des Einsatzstoffgemisches gesteigert wird. Das Verfahren soll im industriellen Format wirtschaftlich betrieben werden können. Insbesondere soll eine bestehende Oxo-Anlage zur besseren Rohstoffausnutzung ertüchtigt werden.

Gelöst wird diese Aufgabe durch eine Kombination einer Hydroformylierung und einer Dehydrierung, wobei die Besonderheit darin besteht, dass die Dehydrierung stromabwärts hinter der Hydroformylierung angeordnet ist und dabei deutlich kleiner dimensioniert ist als herkömmliche Dehydrierungen, die stromaufwärts vorgesehen sind. Eine geschickte Produktabtrennung entfernt effektiv im Prozess gebildete Störstoffe.

Gegenstand der Erfindung ist konkret ein Verfahren zur Herstellung von n-Pentanal mit den folgenden Schritten:
a) Bereitstellen eines Einsatzstoffgemisches, welches die folgende, sich zu 100 Gew.-% ergänzende Zusammensetzung aufweist:

| | |
|---|---|
| • n-Butan: | 70 Gew.-% bis 90 Gew.-%; |
| • n-Buten: | 10 Gew.-% bis 30 Gew.-%; |
| • 1-Buten: | 0 Gew.-% bis 3 Gew.-%; |
| • Isobuten: | 0 Gew.-% bis 3 Gew.-%; |
| • Isobutan: | 0 Gew.-% bis 3 Gew.-%; |
| • 1.3-Butadien: | 0 Gew.-% bis 1 Gew.-%; |
| • sonstige Stoffe: | 0 Gew.-% bis 1 Gew.-%; |

b) Vermischen des Einsatzstoffgemisches mit einem Rezyklat unter Erhalt einer Beschickung;
c) Beaufschlagen der Beschickung mit Kohlenmonoxid und Wasserstoff in Gegenwart eines ersten Katalysatorsystems zwecks Umsetzung zumindest eines Teils des in der Beschickung enthaltenden n-Butens in Aldehyde im Wege der Hydroformylierung, wodurch ein Hydroformylierungsgemisch erhalten wird;
d) Gewinnen einer Primärproduktfraktion aus dem Hydroformylierungsgemisch, wobei die Primärproduktfraktion die folgende, sich zu 100 Gew.-% ergänzende Zusammensetzung aufweist:

| | |
|---|---|
| • n-Pentanal: | 90 Gew.-% bis 98.5 Gew.-%; |
| • 2-Methylbutanal: | 0 Gew.-% bis 5 Gew.-%; |
| • 3-Methylbutanal: | 0 Gew.-% bis 3 Gew.-%; |
| • sonstige Stoffe: | 0 Gew.-% bis 2 Gew.-%; |

e) Gewinnen einer Nebenfraktion aus dem Hydroformylierungsgemisch, wobei die Nebenfraktion die folgende, sich zu 100 Gew.-% ergänzende Zusammensetzung aufweist:

| | |
|---|---|
| • n-Butan: | 80 Gew.-% bis 92 Gew.-%; |
| • n-Buten: | 8 Gew.-% bis 20 Gew.-%; |
| • sonstige Stoffe: | 0 Gew.-% bis 1 Gew.-%; |

f) Unterwerfen der Nebenfraktion einer Dehydrierung in Gegenwart eines zweiten Katalysatorsystems unter Erhalt eines Dehydrierungsgemisches, welches die folgende, sich zu 100 Gew.-% ergänzende Zusammensetzung aufweist:

| | |
|---|---|
| • n-Buten: | 50 Gew.-% bis 60 Gew.-%; |
| • n-Butan: | 40 Gew.-% bis 50 Gew.-%; |
| • Methan: | 0 Gew.-% bis 4 Gew.-%; |
| • Ethen: | 0 Gew.-% bis 3 Gew.-%; |
| • Propen: | 0 Gew.-% bis 2 Gew.-%; |
| • 1.3-Butadien: | 0 Gew.-% bis 3 Gew.-%; |
| • sonstige Stoffe: | 0 Gew.-% bis 1 Gew.-%; |

g) Unterwerfen des Dehydrierungsgemisches einer selektiven Hydrierung in Gegenwart eines dritten Katalysatorsystems unter Erhalt eines Hydrierungsgemisches, welches die folgende, sich zu 100 Gew.-% ergänzende Zusammensetzung aufweist:

| | |
|---|---|
| • n-Buten: | 50 Gew.-% bis 60 Gew.-%; |
| • n-Butan: | 40 Gew.-% bis 50 Gew.-%; |
| • 1.3-Butadien: | 0 Gew.-ppm bis 500 Gew.-ppm; |
| • sonstige Stoffe: | 0 Gew.-% bis 5 Gew.-%; |

h) Unmittelbares Verwenden des Hydrierungsgemisches als Rezyklat, oder
Aufreinigen des Hydrierungsgemisches unter Erhalt des Rezyklats,
wobei die Dehydrierung ohne Zugabe eines Oxidationsmittels erfolgt
oder
wobei die Dehydrierung mit Zugabe von Sauerstoff erfolgt, wobei die zugegebene Menge Sauerstoff bezogen auf die Masse des in der Nebenfraktion enthaltenden n-Butans 1.4 Gew.-% bis 14 Gew.-% beträgt.

Die Erfindung beruht auf der Erkenntnis, dass es mit unerwartet geringem Aufwand möglich ist, das in der Hydroformylierung nicht umsetzbare n-Butan als Nebenfraktion zu gewinnen, es zu dehydrieren, und die dadurch erhaltenen Butene zurück in die Hydroformylierung zu rezyklieren, um sie dort in die gewünschten Aldehyde zu überführen. Damit werden die im Einsatzstoffgemisch enthaltenen C-Atome sehr effizient ausgenutzt. Überraschend dabei ist, dass die in der Dehydrierung in nicht unwesentlichen Mengen anfallenden Nebenprodukte (diese nehmen in der Regel ca. 8 Gew.-% des Austrags der Dehydrierung ein) sich mit ohnehin vorhandenen Trenneinrichtungen entfernen lassen, weswegen der Mehraufwand zur Störstoffbeseitigung gering ausfällt. Die Hydroformylierung reagiert nämlich nur auf wenige, in einer stromabwärts angeordneten Dehydrierung entstehenden, Nebenprodukte empfindlich und kann aus einem Teil davon neben dem n-Pentanal sogar zusätzliche Wertprodukte bilden. Im Ergebnis ist der Prozess damit wirtschaftlich, obgleich die Dehydrierung einen Mehrbedarf an Energie bedeutet. Da die Dehydrierung vergleichsweise klein dimensioniert ist, kann ihr Energiebedarf nämlich zumindest teilweise mit Überschussenergie aus anderen Prozessen gedeckt werden. Dazu später mehr.

Wie bereits erwähnt ist die Dehydrierung energieintensiv. Die Effizienz dieses Prozesses hängt somit stark von dem eingesetzten Katalysatorsystem ab. Bei dem zweiten Katalysatorsystem, welches für die Dehydrierung eingesetzt wird, handelt es sich vorzugsweise um einen Feststoff, welcher Platin, Zinn und Aluminiumoxid enthält. Darüber hinaus können weitere katalytisch aktive Materialien wie beispielsweise Zink und/oder Calcium enthalten sein. Insbesondere ist das Al₂O₃ mit Zn und/oder Ca modifiziert. Entsprechende Katalysatoren werden oft als Pt/Zn-Systeme bezeichnet und sind US4152365, US4926005 und US5151401 zu entnehmen. Die Dehydrierung kann in dessen Gegenwart in der Gasphase, bei einem Druck von 0.8*10⁵ Pa bis 1.2*10⁵ Pa und einer Temperatur von 450°C bis 700°C erfolgen. Die Dehydrierung ist mithin heterogen katalysiert, was eine aufwändige Abtrennung des zweiten Katalysatorsystems von dem Dehydrierungsgemisch entbehrlich macht.
Vorzugsweise findet die Dehydrierung bei einer vergleichsweise geringen Temperatur zwischen 450°C und 530°C statt, was Energie spart. Das dabei erzielte Produktspektrum ist für den hier benötigten Zweck passend. Auch bei dieser vergleichsweise kalten Dehydrierung sollten die Drücke zwischen 0.8*10⁵ Pa bis 1.2*10⁵ Pa liegen.

Da der Katalysator durch Koksablagerungen mit der Zeit desaktiviert wird, muss er regelmäßig regeneriert oder ausgetauscht werden. Dies wird dadurch erleichtert, dass für die Dehydrierung mindestens zwei jeweils beheizte und jeweils mit dem zweiten Katalysatorsystem befüllte Reaktoren vorgesehen sind, wobei die Reaktoren wahlweise einzeln oder gleichzeitig parallel und/oder seriell mit Nebenfraktion beaufschlagbar sind. Auf diese Weise kann stets ein Reaktor abgeschaltet und der darin enthaltene Dehydrier-Katalysator ausgebaut bzw. regeneriert werden, währenddessen der andere Reaktor weiter läuft. Somit kann der Prozess kontinuierlich gefahren werden. Die Regeneration erfolgt durch Spülen mit (vorzugsweise heißer) Luft oder Wasserdampf, wodurch der Koks abgebrannt wird. Die Regeneration erfolgt vorzugsweise in situ, also ohne Ausbau aus dem Reaktor.

Eine besonders bevorzugte Weiterbildung der Erfindung sieht vor dass die Dehydrierung in einem Reaktor betrieben wird, der elektrisch beheizt ist. Unter einer elektrischen Heizung ist ebenso eine Ohm'sche Widerstandsheizung zu verstehen wie ein induktiv beheizter Reaktor.

Eine elektrisch beheizte Dehydrierung ist ungewöhnlich weil derartige Reaktoren üblicherweise mit Brenngas beheizt werden. Die elektrische Beheizung ist deswegen möglich, weil die hier eingesetzte Dehydrierung vergleichsweise klein ist. Die elektrische Beheizung hat den entscheidenden Vorteil, dass sie sich mit überschüssiger elektrischer Energie betreiben lässt, wie sie etwa aus erneuerbaren Energiequellen anfällt. So kann die Dehydrierung etwa gezielt dann betrieben werden, wenn aufgrund der gegenwärtigen Wetterlage viel Strom aus Wind- oder Sonnenkraft anfällt, dieser aber nicht im Netz nachgefragt wird. Auf diese Weise kann die Anlage gezielt negative Regelleistung für das Stromnetz anbieten.

Die Selektivhydrierung dient der Unschädlichmachung von Störstoffen, die in der Dehydrierung entstehen, so zum Beispiel mehrfach ungesättigte Kohlenwasserstoffe wie 1.3-Butadien. Die wertvollen Alkene dürfen dabei nicht hydriert werden. Die Selektivhydrierung erfolgt in der Flüssigphase bei einem Druck von 18*10⁵ Pa bis 22*10⁵ Pa und bei einer Temperatur von 40°C bis 80°C. Als Katalysator wird Festbettkatalysator eingesetzt, der 0.1 bis 2 Massen-% Palladium und ein Trägermaterial (Aktivkohle oder Aluminiumoxid) aufweist. Die Selektivhydrierung erfolgt in Gegenwart von 0.05 bis 10 Massen-ppm Kohlenmonoxid, bezogen auf die Masse des Dehydrierungsgemisches. Das Kohlenmonoxid dient als Moderator und kann aus der Dehydrierung selbst stammen. Auf diese Weise bleiben die Alkene in der Selektivhydrierung erhalten.

Anders als die Dehydrierung erfolgt die Selektivhydrierung in der flüssigen Phase. Deswegen muss das Dehydrierungsgemisch vor der Selektivhydrierung verflüssigt werden. Die Verflüssigung erfolgt durch Kompression und Kühlung. Die bei der Kühlung gewonnene Wärme kann zum Vorwärmen der Nebenfraktion vor der Dehydrierung verwendet werden. Das spart Energie. Es ist thermodynamisch vorteilhaft, die Kühlung als eine zwischen den Verdichtungsstufen angeordnete Zwischenkühlung auszuführen.

Alternativ zu dem Pt/Sn-Systemen kann für die Dehydrierung auch ein Feststoff eingesetzt werden, welcher Aluminiumoxid und Chromoxid enthält. Solche sogenannte Chromia/Alumina-Katalysatoren sind in US3665049 bzw. US3778388 offenbart. Die Dehydrierung erfolgt dann in der Gasphase, bei einem Druck von 0.8*10⁵ Pa bis 1.2*10⁵ Pa und einer Temperatur von 600°C bis 700°C. Die Ausführungen zu dem Pt/Sn -System gelten für Chromia/Alumina-Katalysatoren entsprechend.

Ein weiteres für die Dehydrierung geeignetes Katalysatorsystem enthält Aluminiumoxid und Magnesio-Chromit. Ein Beispiel ist Finocchio et al. Catalysis Today 28 (1996) 381-389 zu entnehmen. Mit diesem Katalysator erfolgt die Dehydrierung in der Gasphase, bei einem Druck von 0.8*10⁵ Pa bis 1.2*10⁵ Pa und einer Temperatur von 600°C bis 700°C.

Neben den genannten heterogenen Systemen kann die Dehydrierung auch homogen katalysiert werden. Dies hat den Vorteil dass die Dehydrierung in der Flüssigphase erfolgen kann, was die Prozessintensität steigert und die Verflüssigung vor der Selektivhydrierung erübrigt. Bei dem zweiten Katalysatorsystem handelt es sich dann um eine im Reaktionsgemisch der Dehydrierung gelöste metallorganische Verbindung.

Die metallorganische Verbindung kann Iridium als Zentralatom umfassen an welchem mindestens ein Pincer-Ligand komplexiert ist. Die Dehydrierung würde dann bei einer Temperatur von 100°C bis 250°C und bei einem Druck von 800*10⁵ Pa bis 1200*10⁵ Pa erfolgen. Ein solches Verfahren ist in WO2014192020A2 beschrieben.

Alternativ kann es sich beider metallorganischen Verbindung um [Rh(PMe3)2(CO)Cl]2 handeln. Dies ist ein Photokatalysator, welcher die Dehydrierung unter Einwirkung von UV Strahlung ermöglicht. Dies ist besonders nachhaltig, da als Energiequelle Sonnenlicht genutzt werden kann:
Chowdhury, A. D., Weding, N., Julis, J., Franke, R., Jackstell, R. and Beller, M. (2014), Towards a Practical Development of Light-Driven Acceptorless Alkane Dehydrogenation. Angew. Chem. Int. Ed., 53: 6477-6481. doi:10.1002/anie.201402287

Gemäß der Erfindung erfolgt die Dehydrierung grundsätzlich ohne Zugabe eines Oxidationsmittels wie Sauerstoff. Es handelt sich somit nicht um eine oxidative Dehydrierung (ODH).

Gleichwohl kann es vorteilhaft sein, eine geringe Menge an Sauerstoff in die Dehydrierung hinzuzugeben, denn dadurch lassen sich nämlich im laufenden Betrieb Koksablagerungen von dem Katalysator entfernen. Die dabei entstehende Wärme kommt der endothermen Dehydrierung zu Gute.

Eine "geringe Menge Sauerstoff" bedeutet in diesem Zusammenhang eine Sauerstoffmenge von 1.4 Gew.-% bis 14 Gew.-% bezogen auf die Masse an n-Butan enthalten in der Nebenfraktion. Diese Sauerstoffzugabe ist deutlich geringer als bei einer herkömmlichen ODH.

In einer bevorzugten Ausführungsform der Erfindung wird das Hydrierungsgemisch ohne Aufreinigung als Rezyklat mit dem Einsatzstoffgemisch vermischt. Dies erspart Investitionskosten setzt aber voraus, dass die Selektivhydrierung sämtliche die Hydroformylierung störende Nebenprodukte der Dehydrierung neutralisiert.

Idealerweise wird das Hydroformylierungsgemisch ausschließlich getrennt in die Primärproduktfraktion und die Nebenfraktion. Dies ist dann möglich, wenn außer dem n-Buten keine Komponenten mit einem geringen Siedepunkt als n-Buten in der Dehydrierung anfallen.

In der Praxis wird die Dehydrierung aber C₁-bis C₃-Kohlenwasserstoffe bilden, etwa Methan, Ethen und Propen. Diese Tatsache erfordert eine Auftrennung des Hydroformylierungsgemisches in eine Leichtsiederfraktion, die Nebenfraktion und die Primärproduktfraktion. In der Leichtsiederfraktion finden sich dann die C₁- bis C₃-Kohlenwasserstoffe.

In einer solchen Konstellation bietet es sich an, dass Hydroformylierungsgemisch aufzutrennen in die Leichtsiederfraktion, die Nebenfraktion, die Primärproduktfraktion und in eine Sekundärproduktfraktion, wobei die Sekundärproduktfraktion die folgende, sich zu 100 Gew.-% ergänzende Zusammensetzung aufweist:

| | |
|---|---|
| • Propanal: | 50 Gew.-% bis 70 Gew.-%; |
| • n-Butanal: | 30 Gew.-% bis 50 Gew.-%; |
| • sonstige Stoffe: | 0 Gew.-% bis 10 Gew.-%. |

Das in der Dehydrierung gebildete Ethen und Propen wird nämlich in der Hydroformylierung in die entsprechende C₃- bzw. C₄-Aldehyde umgesetzt. Diese Aldehyde stellen neben dem n-Pentanal weitere Wertprodukte dar, die als Sekundärproduktfraktion gewonnen werden. Die an sich unerwünschten Nebenprodukte der Dehydrierung (Ethen, Propen) können so gewinnbringend genutzt werden. Dass die gemeinschaftliche Hydroformylierung mehrerer Substrate funktioniert, zeigt WO2015/132068A1.

Ein Bonuseffekt der Bildung von C₃- bzw. C₄-Aldehyden besteht darin, dass diese etwaig entstehendes Reaktionswasser in einem Azeotrop binden und so aus dem Prozess ausschleusen. Eine separate Wasserabtrennung wird dadurch entbehrlich.

Da die Dehydrierung im erfindungsgemäßen Prozess stromabwärts hinter der Hydroformylierung angeordnet ist (also in Richtung der Wertschöpfungskette) kommt sie mit einer deutlich geringeren Produktionskapazität aus als eine kommerziell angebotene Dehydrierung. Für die Anordnung hinter einer Oxo-Anlage im heute industrieüblichen Maßstab genügt es, wenn die Dehydrierung apparativ zur kontinuierlichen Verarbeitung eines Massenstroms der Nebenfraktion von kleiner 4kg/s ausgelegt ist. Diese Dimensionierung entspricht im kontinuierlichen Betrieb (8000h im Jahr) einer Anlagenkapazität von 120 kt/a, etwa einem Fünftel der heute üblichen Größe. Eine kommerziell erhältliche Dehydrieranlage kann mithin nicht verwendet werden, da sie überdimensioniert und unwirtschaftlich wäre.

Sollte das in der Hydroformylierung nicht umgesetzte Buten das in der Dehydrierung eingesetzte zweite Katalysatorsystem zu stark verkoken, bietet es sich an, das Butan/Buten-Gemisch vor der Dehydrierung zu hydrieren. In diesem Fall würde die Nebenfraktion durch Destillation mit anschließender Hydrierung gewonnen.

Ein besonderer Vorteil des hier beschriebenen Verfahrens ist, dass es nicht nur auf der "grünen Wiese" neu errichtet werden kann, sondern dass es auch möglich ist, eine bestehende Oxo-Anlage für C₄-Hydroformylierung um eine entsprechend kleine Dehydrierung zu ergänzen, wodurch mit geringem Kapitaleinsatz die Stoffeffizienz der Anlage gesteigert werden kann.

Gegenstand der Erfindung ist mithin auch die Verwendung einer Anlage zur Dehydrierung von Alkanen, aufweisend mindestens einen beheizten Reaktor, welcher mit einem zweiten Katalysatorsystem gefüllt ist, zum Nachrüsten einer bestehenden Anlage zur Herstellung von n-Pentanal aus Einsatzstoffgemischen enthaltend n-Buten und n-Butan im Wege der Hydroformylierung, indem die Anlage zur Dehydrierung stromabwärts der Anlage zur Hydroformylierung angeordnet, mit einer Nebenfraktion der Hydroformylierung gespeist und der Austrag der Dehydrierung mit oder ohne Aufreinigung in die Hydroformylierung zurückgeführt wird.

Das erfindungsgemäße Verfahren soll nun anhand von Prozessfließbildern erläutert werden. Diese zeigen vereinfachend:
- Figur 1:: Verfahrensschaubild Grundkonzept;
- Figur 2:: wie Figur 1 zusätzlich mit Abtrennung Sekundärprodukt;
- Figur 3:: wie Figur 1 zusätzlich mit Hydrierung vor Dehydrierung.

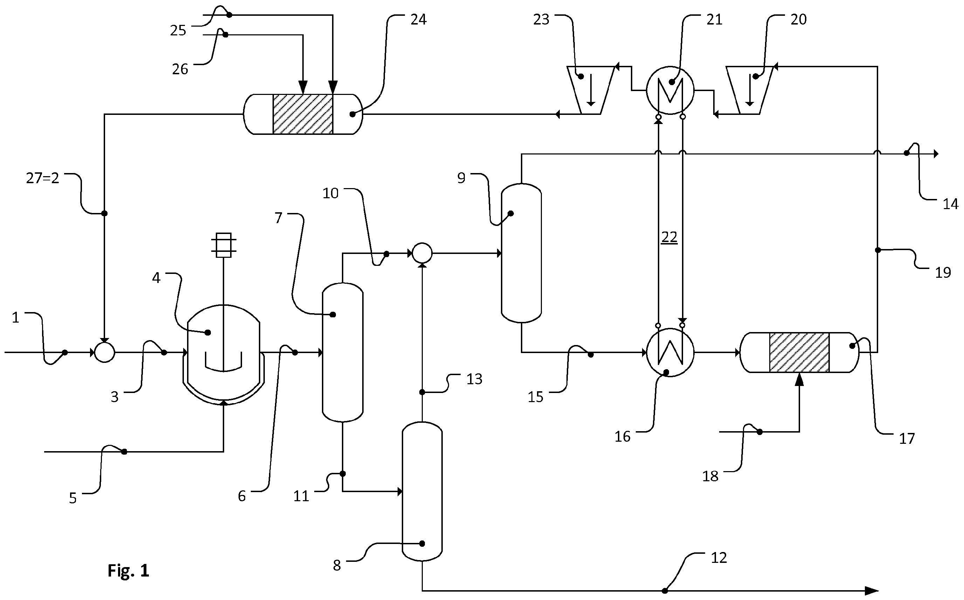

Das Grundkonzept des erfindungsgemäßen Verfahrens ist in Figur 1 dargestellt. Ein von außerhalb des Prozesses bezogenes Einsatzstoffgemisch 1 enthaltend überwiegend n-Butan sowie eine Restmenge n-Buten wird mit einem Rezyklat 2 zu einer Beschickung 3 vermischt. Das Rezyklat stammt aus dem Prozess selbst, dazu später mehr.

Die Beschickung 3 wird in eine Hydroformylierung 4 gefahren und dort zusammen mit Synthesegas 5 (das ist eine Mischung aus Kohlenmonoxid und Wasserstoff) in üblicher Weise umgesetzt. Aus der Hydroformylierung 4 wird ein Hydroformylierugsgemisch 6 abgezogen, welches das gewünschte n-Pentanal (entstanden aus der Umsetzung von n-Buten mit Synthesegas), weitere Nebenprodukte nicht umgesetztes n-Buten und vor allem nicht umgesetztes n-Butan enthält. Die notwendige Abscheidung des in der Hydroformylierung 4 verwendeten, homogenen ersten Katalysatorsystems ist hier nicht dargestellt.

In einer Trennsequenz umfassend drei Destillationskolonnen 7, 8, 9 wird das Hydroformylierungsgemisch destillativ aufgetrennt. Dafür wird das Hydroformylierungsgemisch 6 in die erste Kolonne 7 gefahren und in Kopfprodukt 10 und Sumpfprodukt 11 getrennt. Mit dem Sumpfprodukt 11 der ersten Kolonne 7 wird die zweite Kolonne 8 gespeist. Am Sumpf der zweiten Kolonne wird eine Primärproduktfraktion 12 gewonnen, welche das aufgereinigte n-Pentanal enthält.

Das Kopfprodukt 13 der zweiten Kolonne 8 wird mit dem Kopfprodukt 10 der ersten Kolonne 7 vermischt und in die dritte Kolonne 9 gefahren. An deren Kopf wird eine Leichtsiederfraktion 14 abgezogen, am Sumpf eine Nebenfraktion 15.

Die Nebenfraktion enthält im Wesentlichen das nicht hydroformylierbare n-Butan sowie einen nennenswerten Anteil an n-Buten, welcher in der Hydroformylierung 4 nicht umgesetzt wurde.

Um die in der Nebenfraktion 15 enthaltenen C-Atome für das Verfahren nutzbar zu machen, wird die Nebenfraktion 15 zunächst in einem ersten Wärmetauscher 16 vorgewärmt und sodann in einer Dehydrierung 17 katalytisch dehydriert. Die Dehydrierung 17 erfolgt in der Gasphase in Gegenwart eines heterogenen zweiten Katalysatorsystems, optional unter Zugabe von geringen Mengen Sauerstoff 18.
Die Dehydrierung erfordert thermische Energie, die vorzugsweise elektrisch erzeugt wird. Selbstverständlich ist auch eine traditionelle Beheizung mit Brenngas möglich.

Im Zuge Dehydrierung wird das in der Nebenfraktion 15 enthaltende n-Butan in n-Buten umgesetzt. Weitere Stoffe entstehen, wie etwa 1.3-Butadien, Methan, Ethen, Propen. Das diese Stoffe enthaltende Dehydrierungsgemisch 19 wird aus der Dehydrierung gasförmig abgezogen und sodann in einer ersten Verdichterstufe 20 verdichtet. Die dabei anfallende Verdichterwärme wird von einem zweiten Wärmetauscher 21 abgeführt und das Dehydrierungsgemisch 19 dadurch zwischengekühlt. Die in der Zwischenkühlung anfallende Wärme wird zum Vorheizen der Nebenfraktion 15 vor dem Eintritt in die Dehydrierung 17 genutzt. Zu diesem Zwecke sind der erste Wärmetauscher 16 und der zweite Wärmetauscher 21 über einen ein Wärmeträgermedium führenden Kreislauf 22 miteinander verbunden. Die endgültige Verflüssigung des Dehydrierungsgemisch 19 erfolgt in einer zweiten Verdichterstufe 23.

Das nunmehr flüssige Dehydrierungsgemisch wird nunmehr einer Selektivhydrierung 24 unterzogen, in Gegenwart eines heterogenen dritten Katalysatorsystems, unter Zugabe von Wasserstoff 25 und Kohlenmonoxid 26 als Moderator. Durch die Selektivhydrierung 24 werden unerwünschte mehrfach ungesättigte Verbindungen wie 1.3-Butadien hydriert und dadurch neutralisiert. Die Alkene bleiben hingegen erhalten.

Das aus der Selektivhydrierung 24 abgezogene Hydrierungsgemisch 27 wird als Rezyklat 2 mit dem Einsatzstoffgemisch 1 vermischt und so letztendlich wieder dem Prozess zur Verfügung gestellt.

Optional kann das Hydrierungsgemisch 27 noch aufgereinigt und dann als Rezyklat 2 mit dem Einsatzstoffgemisch 1 vermischt werden. Dies ist aber nicht bevorzugt und daher nicht dargestellt.

Die erfindungsgemäße Dehydrierung und Rückführung des Rezyklats 2 bewirkt, dass die in der Nebenfraktion 15 enthaltenden Butane dank Dehydrierung als Butene wieder in die Hydroformylierung gelangen und dort in das Primärprodukt n-Pentanal umgesetzt werden können. Die stoffliche Effizienz des Prozesses ist damit gegenüber einer Hydroformylierung ohne Dehydrierung gesteigert.

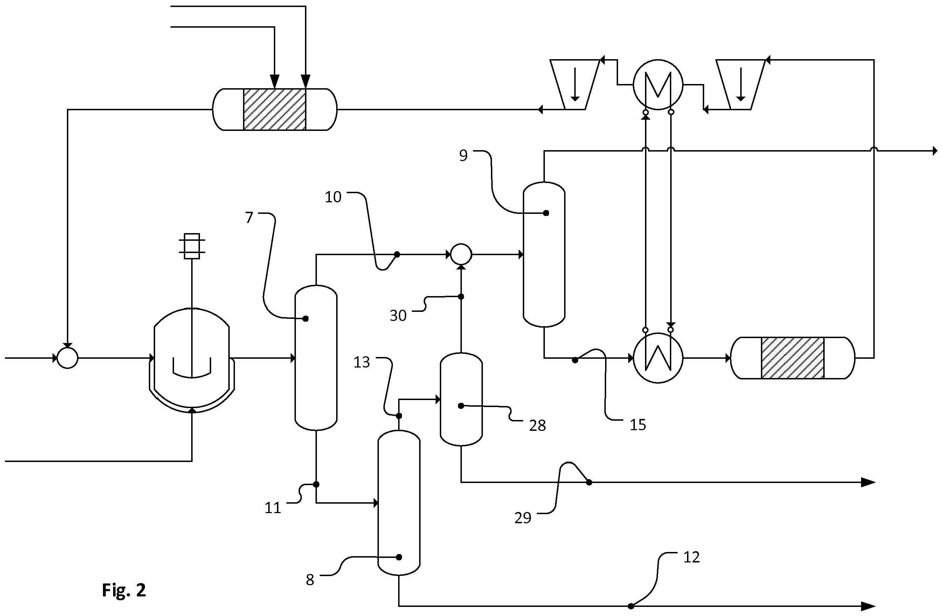

Wie bereits erwähnt, produziert die Dehydrierung 17 neben n-Buten auch Ethen und Propen, beides hydroformylierbare Substrate. Sofern die Bildungsrate von Ethen und Propen groß genug ist, bietet es sich an, in der Trennsequenz eine vierte Kolonne 28 vorzusehen, wie in Figur 2 dargestellt. Die vierte Kolonne 28 wird mit dem Kopfprodukt 13 der zweiten Kolonne 8 gespeist. Vom Sumpf der vierten Kolonne 28 kann dann eine Sekundärproduktfraktion 29 abgezogen werden, enthaltend Propanal und n-Butanal, beides aus Ethen und Propen in der Hydroformylierung 4 gebildet. Das Kopfprodukt 30 der vierten Kolonne 28 wird mit dem Kopfprodukt 10 der ersten Kolonne 7 vermischt und in die dritte Kolonne 9 gefahren.

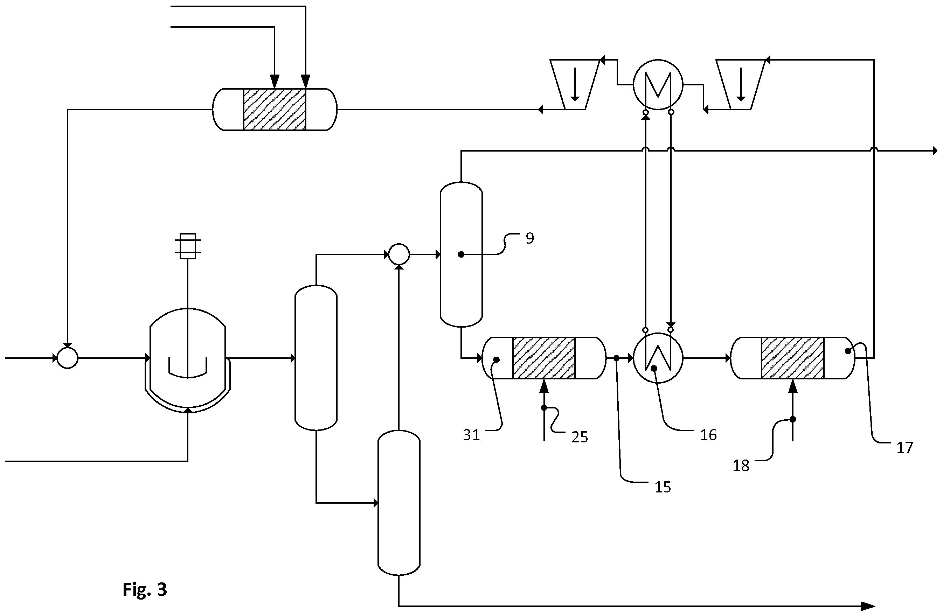

Eine weitere Alternative Ausführungsform zeigt Figur 3. Dort wird die Nebenfraktion 15 dadurch gewonnen, dass das Sumpfprodukt der dritten Kolonne 9 in einer Hydrierung 31 mit Wasserstoff 25 hydriert wird. Diese Maßnahme ist dann erforderlich, wenn der Gehalt an hochreaktiven Stoffen im Sumpfprodukt der dritten Kolone 9 zu groß wäre um dieses direkt in die Dehydrierung 17 zu fahren. Ein solches Vorgehen ist jedoch nicht bevorzugt.

### Bezugszeichenliste

- 1: Einsatzstoffgemisch
- 2: Rezyklat
- 3: Beschickung
- 4: Hydroformylierung
- 5: Synthesegas
- 6: Hydroformylierungsgemisch
- 7: erste Kolonne
- 8: zweite Kolonne
- 9: dritte Kolonne
- 10: Kopfprodukt erste Kolonne
- 11: Sumpfprodukt erste Kolonne
- 12: Primärproduktfraktion
- 13: Kopfprodukt zweite Kolonne
- 14: Leichtsiederfraktion
- 15: Nebenfraktion
- 16: erster Wärmetauscher
- 17: Dehydrierung
- 18: Sauerstoff
- 19: Dehydrierungsgemisch
- 20: erste Verdichterstufe
- 21: zweiter Wärmetauscher
- 22: Kreislauf
- 23: zweite Verdichterstufe
- 24: Selektivhydrierung
- 25: Wasserstoff
- 26: Kohlenmonoxid
- 27: Hydrierungsgemisch
- 28: vierte Kolone
- 29: Sekundärproduktfraktion
- 30: Kopfprodukt der vierten Kolonne
- 31: Hydrierung

## Patentansprüche

1. Verfahren zur Herstellung von n-Pentanal mit den folgenden Schritten:
a) Bereitstellen eines Einsatzstoffgemisches, welches die folgende, sich zu 100 Gew.-% ergänzende Zusammensetzung aufweist:
| | |
|---|---|
| • n-Butan: | 70 Gew.-% bis 90 Gew.-%; |
| • n-Buten: | 10 Gew.-% bis 30 Gew.-%; |
| • 1-Buten: | 0 Gew.-% bis 3 Gew.-%; |
| • Isobuten: | 0 Gew.-% bis 3 Gew.-%; |
| • Isobutan: | 0 Gew.-% bis 3 Gew.-%; |
| • 1.3-Butadien: | 0 Gew.-% bis 1 Gew.-%; |
| • sonstige Stoffe: | 0 Gew.-% bis 1 Gew.-%; |
b) Vermischen des Einsatzstoffgemisches mit einem Rezyklat unter Erhalt einer Beschickung;
c) Beaufschlagen der Beschickung mit Kohlenmonoxid und Wasserstoff in Gegenwart eines ersten Katalysatorsystems zwecks Umsetzung zumindest eines Teils des in der Beschickung enthaltenden n-Butens in Aldehyde im Wege der Hydroformylierung, wodurch ein Hydroformylierungsgemisch erhalten wird;
d) Gewinnen einer Primärproduktfraktion aus dem Hydroformylierungsgemisch, wobei die Primärproduktfraktion die folgende, sich zu 100 Gew.-% ergänzende Zusammensetzung aufweist:
| | |
|---|---|
| • n-Pentanal: | 90 Gew.-% bis 98.5 Gew.-%; |
| • 2-Methylbutanal: | 0 Gew.-% bis 5 Gew.-%; |
| • 3-Methylbutanal: | 0 Gew.-% bis 3 Gew.-%; |
| • sonstige Stoffe: | 0 Gew.-% bis 2 Gew.-%; |
e) Gewinnen einer Nebenfraktion aus dem Hydroformylierungsgemisch, wobei die Nebenfraktion die folgende, sich zu 100 Gew.-% ergänzende Zusammensetzung aufweist:
| | |
|---|---|
| • n-Butan: | 80 Gew.-% bis 92 Gew.-%; |
| • n-Buten: | 8 Gew.-% bis 20 Gew.-%; |
| • sonstige Stoffe: | 0 Gew.-% bis 1 Gew.-%; |
f) Unterwerfen der Nebenfraktion einer Dehydrierung in Gegenwart eines zweiten Katalysatorsystems unter Erhalt eines Dehydrierungsgemisches, welches die folgende, sich zu 100 Gew.-% ergänzende Zusammensetzung aufweist:
| | |
|---|---|
| • n-Buten: | 50 Gew.-% bis 60 Gew.-%; |
| • n-Butan: | 40 Gew.-% bis 50 Gew.-%; |
| • Methan: | 0 Gew.-% bis 4 Gew.-%; |
| • Ethen: | 0 Gew.-% bis 3 Gew.-%; |
| • Propen: | 0 Gew.-% bis 2 Gew.-%; |
| • 1.3-Butadien: | 0 Gew.-% bis 3 Gew.-%; |
| • sonstige Stoffe: | 0 Gew.-% bis 1 Gew.-%; |
g) Unterwerfen des Dehydrierungsgemisches einer selektiven Hydrierung in Gegenwart eines dritten Katalysatorsystems unter Erhalt eines Hydrierungsgemisches, welches die folgende, sich zu 100 Gew.-% ergänzende Zusammensetzung aufweist:
| | |
|---|---|
| • n-Buten: | 50 Gew.-% bis 60 Gew.-%; |
| • n-Butan: | 40 Gew.-% bis 50 Gew.-%; |
| • 1.3-Butadien: | 0 Gew.-ppm bis 500 Gew.-ppm; |
| • sonstige Stoffe: | 0 Gew.-% bis 5 Gew.-%; |
h) Unmittelbares Verwenden des Hydrierungsgemisches als Rezyklat, oder
Aufreinigen des Hydrierungsgemisches unter Erhalt des Rezyklats,
wobei die Dehydrierung ohne Zugabe eines Oxidationsmittels erfolgt
oder
wobei die Dehydrierung mit Zugabe von Sauerstoff erfolgt, wobei die zugegebene Menge Sauerstoff bezogen auf die Masse des in der Nebenfraktion enthaltenden n-Butans 1.4 Gew.-% bis 14 Gew.-% beträgt.

2. Verfahren nach Anspruch 1, **dadurch gekennzeichnet, dass** es sich bei dem zweiten Katalysatorsystem um einen Feststoff handelt, welcher zumindest Platin, Zinn und Aluminiumoxid enthält, und dass die Dehydrierung in der Gasphase, bei einem Druck von 0.8*10⁵ Pa bis 1.2*10⁵ Pa und einer Temperatur von 450°C bis 700°C erfolgt.

3. Verfahren nach Anspruch 1 oder 2, **dadurch gekennzeichnet, dass** für die Dehydrierung mindestens zwei jeweils beheizte und jeweils mit dem zweiten Katalysatorsystem befüllte Reaktoren vorgesehen sind, wobei die Reaktoren wahlweise einzeln oder gleichzeitig parallel und/oder seriell mit Nebenfraktion beaufschlagbar sind.

4. Verfahren nach Anspruch 3, **dadurch gekennzeichnet, dass** die Reaktoren elektrisch beheizt sind.

5. Verfahren nach Anspruch 1 bzw. einem der Ansprüche 2 bis 4, **dadurch gekennzeichnet, dass** das Dehydrierungsgemisch durch Kompression und Kühlung verflüssigt wird und die Selektivhydrierung in der Flüssigphase bei einem Druck von 18*10⁵ Pa bis 22*10⁵ Pa und einer Temperatur von 40°C bis 80°C erfolgt.

6. Verfahren nach Anspruch 5, **dadurch gekennzeichnet, dass** die bei der Kühlung gewonnene Wärme zum Vorwärmen der Nebenfraktion verwendet wird.

7. Verfahren nach Anspruch 5 oder 6, **dadurch gekennzeichnet, dass** die Kompression in zwei aufeinander folgenden Verdichtungsstufen erfolgt, und dass als Kühlung eine zwischen den Verdichtungsstufen angeordnete Zwischenkühlung vorgesehen ist.

8. Verfahren nach Anspruch 1 bzw. einem der Ansprüche 3 bis 7, **dadurch gekennzeichnet, dass** es sich bei dem zweiten Katalysatorsystem um einen Feststoff handelt, welcher Aluminiumoxid und Chromoxid enthält, und dass die Dehydrierung in der Gasphase, bei einem Druck von 0.8*10⁵ Pa bis 1.2*10⁵ Pa und einer Temperatur von 600°C bis 700°C erfolgt.

9. Verfahren nach Anspruch 1 bzw. einem der Ansprüche 3 bis 7, **dadurch gekennzeichnet, dass** es sich bei dem zweiten Katalysatorsystem um einen Feststoff handelt, welcher Aluminiumoxid und Magnesio-Chromit enthält, und dass die Dehydrierung in der Gasphase, bei einem Druck von 0.8*10⁵ Pa bis 1.2*10⁵ Pa und einer Temperatur von 600°C bis 700°C erfolgt.

10. Verfahren nach Anspruch 1 bzw. einem der Ansprüche 2 bis 9, **dadurch gekennzeichnet, dass** das Hydrierungsgemisch ohne Aufreinigung als Rezyklat mit dem Einsatzstoffgemisch vermischt wird.

11. Verfahren nach Anspruch 1 bzw. einem der Ansprüche 2 bis 10, **dadurch gekennzeichnet, dass** das Hydroformylierungsgemisch ausschließlich getrennt wird in die Primärproduktfraktion und die Nebenfraktion.

12. Verfahren nach Anspruch 1 bzw. einem der Ansprüche 2 bis 10, **dadurch gekennzeichnet, dass** das Hydroformylierungsgemisch getrennt wird in eine Leichtsiederfraktion, die Nebenfraktion und die Primärproduktfraktion.

13. Verfahren nach Anspruch 12, **dadurch gekennzeichnet, dass** das Hydroformylierungsgemisch getrennt wird in die Leichtsiederfraktion, die Nebenfraktion, die Primärproduktfraktion und in eine Sekundärproduktfraktion, wobei die Sekundärproduktfraktion die folgende, sich zu 100 Gew.-% ergänzende Zusammensetzung aufweist:
| | |
|---|---|
| • Propanal: | 50 Gew.-% bis 70 Gew.-%; |
| • n-Butanal: | 30 Gew.-% bis 50 Gew.-%; |
| • sonstige Stoffe: | 0 Gew.-% bis 10 Gew.-%. |

14. Verfahren nach Anspruch 1 bzw. einem der Ansprüche 2 bis 13, **dadurch gekennzeichnet, dass** der Massenstrom der Nebenfraktion kleiner ist als 4 kg/s und die Dehydrierung apparativ zur kontinuierlichen Verarbeitung dieses Massenstroms dimensioniert ist.

15. Verfahren nach Anspruch 1 bzw. einem der Ansprüche 2 bis 14, **dadurch gekennzeichnet, dass** die Nebenfraktion durch Destillation mit anschließender Hydrierung gewonnen wird.

16. Verwendung einer Anlage zur Dehydrierung von Alkanen, aufweisend mindestens einen beheizten Reaktor, welcher mit einem zweiten Katalysatorsystem gefüllt ist, zum Nachrüsten einer bestehenden Anlage zur Herstellung von n-Pentanal aus Einsatzstoffgemischen enthaltend n-Buten und n-Butan im Wege der Hydroformylierung, indem die Anlage zur Dehydrierung stromabwärts der Anlage zur Hydroformylierung angeordnet, mit einer Nebenfraktion der Hydroformylierung gespeist und der Austrag der Dehydrierung mit oder ohne Aufreinigung in die Hydroformylierung zurückgeführt wird.

## Claims

1. Process for producing n-pentanal comprising the following steps of:
a) providing a feedstock mixture having the following composition which sums to 100 wt%:
| | |
|---|---|
| • n-butane: | 70 wt% to 90 wt%; |
| • n-butene: | 10 wt% to 30 wt%; |
| • 1-butene: | 0 wt% to 3 wt%; |
| • isobutene: | 0 wt% to 3 wt%; |
| • isobutane: | 0 wt% to 3 wt%; |
| • 1,3-butadiene: | 0 wt% to 1 wt%; |
| • other substances: | 0 wt% to 1 wt%; |
b) mixing the feedstock mixture with a recyclate to obtain a feed;
c) treating the feed with carbon monoxide and hydrogen in the presence of a first catalyst system to convert at least a portion of the n-butene present in the feed into aldehydes by hydroformylation to obtain a hydroformylation mixture;
d) recovering a primary product fraction from the hydroformylation mixture, wherein the primary product fraction has the following composition which sums to 100 wt%:
| | |
|---|---|
| • n-pentanal: | 90 wt% to 98.5 wt%; |
| • 2-methylbutanal: | 0 wt% to 5 wt%; |
| • 3-methylbutanal: | 0 wt% to 3 wt%; |
| • other substances: | 0 wt% to 2 wt%; |
e) recovering a subsidiary fraction from the hydroformylation mixture, wherein the subsidiary fraction has the following composition which sums to 100 wt%:
| | |
|---|---|
| • n-butane: | 80 wt% to 92 wt%; |
| • n-butene: | 8 wt% to 20 wt%; |
| • other substances: | 0 wt% to 1 wt%; |
f) subjecting the subsidiary fraction to a dehydrogenation in the presence of a second catalyst system to obtain a dehydrogenation mixture having the following composition which sums to 100 wt%:
| | |
|---|---|
| • n-butene: | 50 wt% to 60 wt%; |
| • n-butane: | 40 wt% to 50 wt%; |
| • methane: | 0 wt% to 4 wt%; |
| • ethene: | 0 wt% to 3 wt%; |
| • propene: | 0 wt% to 2 wt%; |
| • 1,3-butadiene: | 0 wt% to 3 wt%; |
| • other substances: | 0 wt% to 1 wt%; |
g) subjecting the dehydrogenation mixture to a selective hydrogenation in the presence of a third catalyst system to obtain a hydrogenation mixture having the following composition which sums to 100 wt%:
| | |
|---|---|
| • n-butene: | 50 wt% to 60 wt%; |
| • n-butane: | 40 wt% to 50 wt%; |
| • 1,3-butadiene: | 0 ppm by weight to 500 ppm by weight; |
| • other substances: | 0 wt% to 5 wt%; |
h) direct use of the hydrogenation mixture as recyclate
or
purification of the hydrogenation mixture to obtain the recyclate,
wherein the dehydrogenation is effected without addition of an oxidant
or
wherein the dehydrogenation is effected with addition of oxygen, wherein the added amount of oxygen based on the mass of the n-butane present in the subsidiary fraction is 1.4 wt% to 14 wt%.

2. Process according to Claim 1, **characterized in that** the second catalyst system is a solid comprising at least platinum, tin and aluminium oxide and that the dehydrogenation is effected in the gas phase at a pressure of 0.8*10⁵ Pa to 1.2*10⁵ Pa and a temperature of 450°C to 700°C.

3. Process according to Claim 1 or 2, **characterized in that** at least two reactors, each heated and each filled with the second catalyst system, are provided for the dehydrogenation and the reactors are chargeable with subsidiary fraction individually or simultaneously in parallel and/or serially as desired.

4. Process according to Claim 3, **characterized in that** the reactors are electrically heated.

5. Process according to Claim 1 and/or any of Claims 2 to 4, **characterized in that** the dehydrogenation mixture is liquefied by compression and cooling and the selective hydrogenation is effected in the liquid phase at a pressure of 18*10⁵ Pa to 22*10⁵ Pa and a temperature of 40°C to 80°C.

6. Process according to Claim 5, **characterized in that** the heat recovered during cooling is used for preheating the subsidiary fraction.

7. Process according to Claim 5 or 6, **characterized in that** the compression is effected in two successive compression stages and that the cooling provided is an intercooling arranged between the compression stages.

8. Process according to Claim 1 and/or any of Claims 3 to 7, **characterized in that** the second catalyst system is a solid comprising aluminium oxide and chromium oxide and that the dehydrogenation is effected in the gas phase at a pressure of 0.8*10⁵ Pa to 1.2*10⁵ Pa and a temperature of 600°C to 700°C.

9. Process according to Claim 1 and/or any of Claims 3 to 7, **characterized in that** the second catalyst system is a solid comprising aluminium oxide and magnesiochromite and that the dehydrogenation is effected in the gas phase at a pressure of 0.8*10⁵ Pa to 1.2*10⁵ Pa and a temperature of 600°C to 700°C.

10. Process according to Claim 1 and/or any of Claims 2 to 9, **characterized in that** the hydrogenation mixture is mixed with the feedstock mixture as a recyclate without purification.

11. Process according to Claim 1 and/or any of Claims 2 to 10, **characterized in that** the hydroformylation mixture is exclusively separated into the primary product fraction and the subsidiary fraction.

12. Process according to Claim 1 and/or any of Claims 2 to 10, **characterized in that** the hydroformylation mixture is separated into a low boiler fraction, the subsidiary fraction and the primary product fraction.

13. Process according to Claim 12, **characterized in that** the hydroformylation mixture is separated into the low boiler fraction, the subsidiary fraction, the primary product fraction and into a secondary product fraction, wherein the secondary product fraction has the following composition which sums to 100 wt%:
| | |
|---|---|
| • propanal: | 50 wt% to 70 wt%; |
| • n-butanal: | 30 wt% to 50 wt%; |
| • other substances: | 0 wt% to 10 wt%. |

14. Process according to Claim 1 and/or any of Claims 2 to 13, **characterized in that** the mass flow of the subsidiary fraction is less than 4 kg/s and the apparatus of the dehydrogenation is of a size configured for continuous processing of this mass flow.

15. Process according to Claim 1 and/or any of Claims 2 to 14, **characterized in that** the subsidiary fraction is recovered by distillation with subsequent hydrogenation.

16. Use of a plant for dehydrogenation of alkanes comprising at least a heated reactor filled with a second catalyst system for retrofitting an existing plant for producing n-pentanal from feedstock mixtures comprising n-butene and n-butane by hydroformylation where the plant for dehydrogenation is arranged downstream of the plant for hydroformylation, said plant for dehydrogenation is fed with a subsidiary fraction from the hydroformylation and the effluent from the dehydrogenation is recycled into the hydroformylation with or without purification.

## Revendications

1. Procédé de fabrication de n-pentanal comprenant les étapes suivantes :
a) la préparation d'un mélange de matières d'entrée, qui présente la composition suivante, qui se complète à 100 % en poids :
- n-butane : 70 % en poids à 90 % en poids ;
- n-butène : 10 % en poids à 30 % en poids ;
- 1-butène : 0 % en poids à 3 % en poids ;
- isobutène : 0 % en poids à 3 % en poids ;
- isobutane : 0 % en poids à 3 % en poids ;
- 1,3-butadiène : 0 % en poids à 1 % en poids ;
- autres matières : 0 % en poids à 1 % en poids ;
b) le mélange du mélange de matières d'entrée avec un produit recyclé pour obtenir une alimentation ;
c) le chargement de l'alimentation avec du monoxyde de carbone et de l'hydrogène en présence d'un premier système catalytique pour la transformation d'au moins une partie du n-butène contenu dans l'alimentation en aldéhydes dans le cadre de l'hydroformylation, un mélange d'hydroformylation étant obtenu ;
d) l'obtention d'une fraction de produit primaire à partir du mélange d'hydroformylation, la fraction de produit primaire présentant la composition suivante, qui se complète à 100 % en poids :
- n-pentanal : 90 % en poids à 98,5 % en poids ;
- 2-méthylbutanal : 0 % en poids à 5 % en poids ;
- 3-méthylbutanal : 0 % en poids à 3 % en poids ;
- autres matières : 0 % en poids à 2 % en poids ;
e) l'obtention d'une fraction secondaire à partir du mélange d'hydroformylation, la fraction secondaire présentant la composition suivante, qui se complète à 100 % en poids :
- n-butane : 80 % en poids à 92 % en poids ;
- n-butène : 8 % en poids à 20 % en poids ;
- autres matières : 0 % en poids à 1 % en poids ;
f) la soumission de la fraction secondaire à une déshydrogénation en présence d'un deuxième système catalytique pour obtenir un mélange de déshydrogénation, qui présente la composition suivante, qui se complète à 100 % en poids :
- n-butène : 50 % en poids à 60 % en poids ;
- n-butane : 40 % en poids à 50 % en poids ;
- méthane : 0 % en poids à 4 % en poids ;
- éthène : 0 % en poids à 3 % en poids ;
- propène : 0 % en poids à 2 % en poids ;
- 1,3-butadiène : 0 % en poids à 3 % en poids ;
- autres matières : 0 % en poids à 1 % en poids ;
g) la soumission du mélange de déshydrogénation à une hydrogénation sélective en présence d'un troisième système catalytique pour obtenir un mélange d'hydrogénation, qui présente la composition suivante, qui se complète à 100 % en poids :
- n-butène : 50 % en poids à 60 % en poids ;
- n-butane : 40 % en poids à 50 % en poids ;
- 1,3-butadiène : 0 ppm en poids à 500 ppm en poids ;
- autres matières : 0 % en poids à 5 % en poids ;
h) l'utilisation directe du mélange d'hydrogénation en tant que produit recyclé
ou
la purification du mélange d'hydrogénation pour obtenir le produit recyclé,
la déshydrogénation ayant lieu sans ajout d'un oxydant ou
la déshydrogénation ayant lieu avec ajout d'oxygène, la quantité d'oxygène ajoutée par rapport à la masse du n-butane contenu dans la fraction secondaire étant de 1,4 % en poids à 14 % en poids.

2. Procédé selon la revendication 1, **caractérisé en ce que** le deuxième système catalytique consiste en un solide, qui contient au moins du platine, de l'étain et de l'oxyde d'aluminium, et **en ce que** la déshydrogénation a lieu dans la phase gazeuse, à une pression de 0,8*10⁵ Pa à 1,2*10⁵ Pa et à une température de 450 °C à 700 °C.

3. Procédé selon la revendication 1 ou 2, **caractérisé en ce qu'**au moins deux réacteurs, chacun chauffés et chacun remplis avec le deuxième système catalytique, sont prévus pour la déshydrogénation, les réacteurs pouvant être chargés avec la fraction secondaire au choix individuellement ou simultanément en parallèle et/ou en série.

4. Procédé selon la revendication 3, **caractérisé en ce que** les réacteurs sont chauffés électriquement.

5. Procédé selon la revendication 1 ou selon l'une quelconque des revendications 2 à 4, **caractérisé en ce que** le mélange de déshydrogénation est liquéfié par compression et refroidissement, et l'hydrogénation sélective a lieu dans la phase liquide à une pression de 18*10⁵ Pa à 22*10⁵ Pa et à une température de 40 °C à 80 °C.

6. Procédé selon la revendication 5, **caractérisé en ce que** la chaleur obtenue lors du refroidissement est utilisée pour le préchauffage de la fraction secondaire.

7. Procédé selon la revendication 5 ou 6, **caractérisé en ce que** la compression a lieu en deux étapes de compression successives, et **en ce qu'**un refroidissement intermédiaire agencé entre les étapes de compression est prévu en tant que refroidissement.

8. Procédé selon la revendication 1 ou selon l'une quelconque des revendications 3 à 7, **caractérisé en ce que** le deuxième système catalytique consiste en un solide qui contient de l'oxyde d'aluminium et de l'oxyde de chrome, et **en ce que** la déshydrogénation a lieu dans la phase gazeuse, à une pression de 0,8*10⁵ Pa à 1,2*10⁵ Pa et à une température de 600 °C à 700 °C.

9. Procédé selon la revendication 1 ou selon l'une quelconque des revendications 3 à 7, **caractérisé en ce que** le deuxième système catalytique consiste en un solide qui contient de l'oxyde d'aluminium et de la magnésio-chromite, et **en ce que** la déshydrogénation a lieu dans la phase gazeuse, à une pression de 0,8*10⁵ Pa à 1,2*10⁵ Pa et à une température de 600 °C à 700 °C.

10. Procédé selon la revendication 1 ou selon l'une quelconque des revendications 2 à 9, **caractérisé en ce que** le mélange d'hydrogénation est mélangé sans purification en tant que produit recyclé avec le mélange de matières d'entrée.

11. Procédé selon la revendication 1 ou selon l'une quelconque des revendications 2 à 10, **caractérisé en ce que** le mélange d'hydroformylation est exclusivement séparé en la fraction de produit primaire et la fraction secondaire.

12. Procédé selon la revendication 1 ou selon l'une quelconque des revendications 2 à 10, **caractérisé en ce que** le mélange d'hydroformylation est séparée en une fraction de composants de point d'ébullition faible, la fraction secondaire et la fraction de produit primaire.

13. Procédé selon la revendication 12, **caractérisé en ce que** le mélange d'hydroformylation est séparé en la fraction de point d'ébullition faible, la fraction secondaire, la fraction de produit primaire et une fraction de produit secondaire, la fraction de produit secondaire présentant la composition suivante, qui se complète à 100 % en poids :
- propanai : 50 % en poids à 70 % en poids ;
- n-butanal : 30 % en poids à 50 % en poids ;
- autres matières : 0 % en poids à 10 % en poids.

14. Procédé selon la revendication 1 ou selon l'une quelconque des revendications 2 à 13, **caractérisé en ce que** le débit massique de la fraction secondaire est inférieur à 4 kg/s et la déshydrogénation est dimensionnée en termes d'équipement pour le traitement continu de ce débit massique.

15. Procédé selon la revendication 1 ou selon l'une quelconque des revendications 2 à 14, **caractérisé en ce que** la fraction secondaire est obtenue par distillation avec hydrogénation ultérieure.

16. Utilisation d'une unité pour la déshydrogénation d'alcanes, comprenant au moins un réacteur chauffé, qui est rempli avec un deuxième système catalytique, pour la mise à niveau d'une unité existante pour la fabrication de n-pentanal à partir de mélanges de matières d'entrée contenant du n-butène et du n-butane dans le cadre de l'hydroformylation, selon laquelle l'unité pour la déshydrogénation est agencée en aval de l'unité pour l'hydroformylation, alimentée avec une fraction secondaire de l'hydroformylation, et la sortie de la déshydrogénation est recyclée avec ou sans purification dans l'hydroformylation.
